**Europäisches Patentamt**

(19) **European Patent Office**

**Office européen des brevets**

(11) Numéro de publication: **0 076 759**
**B1**

(12) # FASCICULE DE BREVET EUROPEEN

(45) Date de publication du fascicule du brevet:
29.05.85

(51) Int. Cl.⁴: **G 01 N 25/44**

(21) Numéro de dépôt: **82401809.7**

(22) Date de dépôt: **04.10.82**

(54) Installation de mesure en continu du pouvoir calorifique d'un gaz.

(30) Priorité: **05.10.81 FR 8118722**

(43) Date de publication de la demande:
**13.04.83 Bulletin 83/15**

(45) Mention de la délivrance du brevet:
**29.05.85 Bulletin 85/22**

(84) Etats contractants désignés:
**BE DE GB IT NL**

(56) Documents cités:
**DE - C - 238 892**
**DE - C - 255 403**
**FR - A - 466 280**
**FR - A - 2 476 842**
**US - A - 3 460 385**

(73) Titulaire: **Office National d'Etudes et de Recherches Aerospatiales (O.N.E.R.A.), 29 Avenue de la Division Leclerc, F-92320 Châtillon-sous-Bagneux (FR)**
Titulaire: **SOCIETE NATIONALE ELF AQUITAINE (PRODUCTION), Tour Aquitaine-La Défense, F-92080 Courbevoie (FR)**

(72) Inventeur: **Calvet, Pierre, 90, rue de Limayrac, F-31500 Toulouse (FR)**
Inventeur: **Platet, Bernard, 41, rue Camille Desmoulin, F-31500 Toulouse (FR)**

(74) Mandataire: **Fort, Jacques et al, CABINET PLASSERAUD 84, rue d'Amsterdam, F-75009 Paris (FR)**

BUNDESDRUCKEREI BERLIN

## Description

La présente invention a pour objet une installation de mesure en continu du pouvoir calorifique d'un gaz combustible, permettant de suivre les variations lentes de ce pouvoir.

On connaît déjà de nombreuses installations destinées à mesurer en continu le pouvoir calorifique ou la chaleur de combustion de gaz combustibles, tels que les gaz provenant de gisements naturels. De telles installations sont notamment utilisées en coopération avec des appareils de mesure du débit circulant dans des gazoducs pour déterminer l'énergie thermique fournie.

Les installations de mesure connues utilisent pour la plupart des détecteurs thermométriques constitués par des transducteurs fournissant un signal électrique. L'emploi de ces transducteurs se traduit par des défauts graves. La nécessité d'un isolement introduit des résistances thermiques dont le vieillissement nuit aux performances et à la fiabilité. La faible valeur des surfaces actives des transducteurs permet difficilement d'atteindre une répartition sur le trajet du flux thermique fournissant une intégration satisfaisante des densités de flux sur toute la surface ce transfert. Ce dernier défaut est particulièrement grave lorsque le flux à mesurer traverse une surface d'échange de grande dimension et présente une répartition de densités hétérogène et aléatoire.

La présente invention vise notamment à fournir une installation de mesure en continu répondant mieux que celles antérieurement connues aux exigences de la pratique, notamment en ce qu'elle autorise une intégration satisfaisante du flux sur toute la surface de transfert et une mesure de précision satisfaisante avec des moyens relativement simples.

Dans ce but, l'invention propose notamment une installation de mesure en continu du pouvoir calorifique d'un gaz combustible, comportant un calorimètre dans lequel est disposée une cellule ouverte dont la partie basse entoure un brûleur et des moyens pour alimenter le brûleur et la cellule respectivement en gaz combustible et en air de combustion à pression et débit déterminés, caractérisée en ce que le calorimètre comprend une paroi externe maintenue à température constante et délimitant, avec la paroi de la cellule, un espace annulaire séparé, au moyen d'une cloison parallèle aux parois, en une enceinte interne et une enceinte externe d'épaisseurs faibles par rapport à leurs autres dimensions, lesdites enceintes étant occupées par un même gaz et associées de façon à constituer un thermomètre différentiel à gaz.

On voit que cette disposition permet d'éviter toute résistance de contact thermique puisque le gaz au contact de la paroi de chaque enceinte a la même température que la paroi de la cellule d'une part, la paroi externe d'autre part. L'épaisseur des enceintes, donc des gaines de gaz qu'elles contiennent, étant faible, le gradient de température dans ces enceintes sera sensiblement normal, c'est-à-dire radial si les enceintes, et donc les parois, sont de révolution autour d'un axe. Les inerties calorifiques mises en jeu sont faibles, de sorte que l'installation est apte à suivre les variations du flux thermique produites par une flamme de gaz naturel dont le débit est constant alors que sa composition est lentement variable.

L'installation peut aisément être munie de moyens d'étalonnage. Ces derniers peuvent comporter une résistance chauffante dont l'effet est substitué à celui du brûleur. Il est toutefois préférable d'utiliser des moyens permettant d'alimenter en alternance le brûleur en gaz combustible dont les caractéristiques sont à mesurer et en un gaz de référence, par exemple en méthane pur lorsque l'installation est destinée à mesurer le pouvoir calorifique de gaz naturel.

Pour éviter que le débit d'air aspiré soit notablement affecté par des variations de pouvoir calorifique du gaz combustible, il suffit pratiquement de réaliser la cellule sous forme d'un conduit surmonté d'une cheminée d'évacuation munie d'une résistance électrique chauffante dégageant une puissance largement supérieure au flux à mesurer; ledit conduit étant muni de chicanes pour freiner le courant d'air entraîné par convection. Ainsi, les variations de flux thermique sont sans incidence réelle et il se crée dans la cheminée d'évacuation un courant de convection naturel pratiquement indépendant du flux à mesurer.

Le thermomètre à gaz constitué à partir des enceintes interne et externe peut être réalisé sous forme d'un pont de mesure pneumatique alimenté par une source de gaz à pression variable suivant une loi périodique alternative. En particulier, on peut utiliser un pont pneumatique de mesure du genre décrit et revendiqué dans la demande de brevet déposée ce jour au nom de l'OFFICE NATIONAL D'ETUDES ET DE RECHERCHES AEROSPATIALES pour »Pont pneumatique de mesure« (FR-A-2 514 128).

L'invention sera mieux comprise à la lecture de la description qui suit d'un mode particulier de réalisation, donné à titre d'exemple non limitatif. La description se réfère aux dessins qui l'accompagnent, dans lesquels:

— la figure 1 est un schéma montrant les composants pricipaux du calorimètre de l'installation, les organes de structure étant représentés en coupe suivant un plan vertical;

— la figure 2 est un schéma montrant la constitution d'un pont pneumatique de mesure associé aux enceintes du calorimètre de la figure 1 pour constituer un thermomètre à gaz.

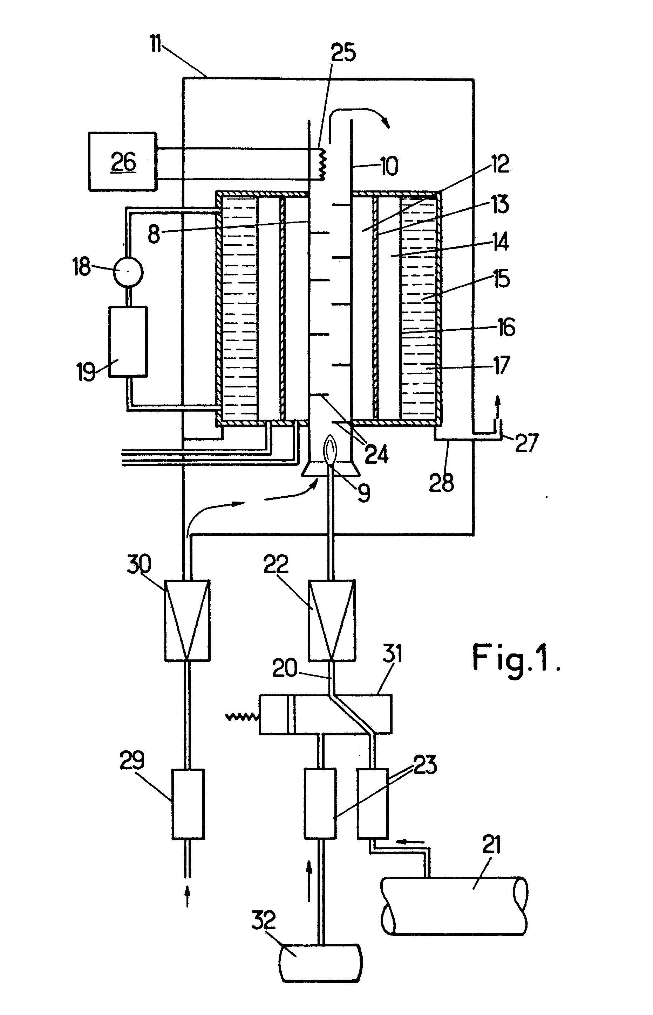

Le calorimètre montré en figure 1 comporte une cellule 8, constituée par un tube qui, lors du fonctionnement, est en position verticale. La partie basse de la cellule entoure le bec d'un brûleur 9 qui reçoit le gaz dont le pouvoir calorifique est à mesurer. La partie haute de la cellule 8 se prolonge par une cheminée d'évacuation 10 dont le rôle apparaîtra plus loin et qui débouche dans une chambre 11 des dimensions nettement supérieures à celles de la cheminée, par laquelle s'effectue l'admission d'air frais et l'évacuation des gaz brûlés à l'abri des courantes d'air.

La cellule 8, de longueur nettement supérieure à son diamètre, est entourée de deux enceintes concentriques 12 et 14, d'épaisseur faible par rapport à leurs autres dimensions, séparées par une cloison 13. La paroi de la cellule 8 sépare les gaz de combustion provenant du brûleur 9 de l'enceinte 12. L'enceinte 14 est délimitée par une paroi externe 16 maintenue à température uniforme et constante par exemple par le liquide 15 occupant un volume annulaire 17 appartenant à un circuit de circulation comprenant une pompe 18 et un thermostat 19. Les enceintes 12 et 14 sont d'épaisseur constante. Elles seront généralement de forme cylindrique, ainsi que les parois et cloison. Les faces en regard de la cellule 8 et de la paroi 16 sont avantageusement absorbantes ou au contraire réfléchissantes au rayonnement infrarouge pour éviter les conséquences du vieillissement sur les propriétés réflectives des corps intermédiaires. La cloison 13 doit être de conductivité moyenne, de façon à ce que l'ensemble 12, 13, 14 constitue un calorimètre à flux dans lequel le gradient thermique principal intervient dans les lames d'air.

L'ensemble constitué par les enceintes 12 et 14, la cloison 13 et les parois de limitation des enceintes constitue un mur thermique dans lequel la transmission de chaleur peut être définie mathématiquement. Le calcul montre que, lorsque les enceintes sont d'épaisseur faible par rapport à leurs dimensions longitudinale et circonférentielle et lorsque les températures varient suffisamment peu dans les lames d'air occupant les enceintes 12 et 14, l'écart de masse entre les deux lames d'air est proportionnel au flux thermique total qui traverse le mur, quelle que soit la répartition de la densité de flux sur les parois. Une mesure de l'écart de masse est donc représentative de l'intégrale de surface des densités de flux, donc du flux total. La mesure de l'écart de masse peut s'effectuer à l'aide de toute disposition équivalente à un thermomètre à gaz dont les enceintes constituent les bulbes. Il est toutefois avantageux d'associer aux enceintes le pont pneumatique de mesure qui sera décrit plus loin.

Le brûler 9 de l'installation doit être alimenté en gaz sous un débit constant ou régulé. Pour cela, on interpose, sur la tubulure 20 d'amenée de gaz au brûleur 9 depuis la conduite 21 dans laquelle s'effectue le prélèvement, un régulateur de pression 22 constitué par exemple par un manodétendeur de précision. Sur le trajet du gaz est de plus inséré un humidificateur 23 de type classique, permettant de saturer le gaz en vapeur d'eau.

Le brûleur fonctionne à pression atmosphérique et des mesures doivent être prises pour éviter que le courant de convection naturel qui s'établit dans la cellule 8 et entraîne un important excédent d'air ne soit pas affecté par les variations du pouvoir calorifique du gaz. Ce résultat peut être atteint en disposant un extracteur rotatif très stable au-dessus de la cellule 8, qui est munie de chicanes 24 destinées à imposer des pertes de charge freinant le courant dû à la convection. Il est toutefois préférable de prévoir une cheminée d'évacuation 10 prolongeant la cellule 8 et munie à sa base d'une résistance électrique chauffante 25 dans laquelle un circuit d'alimentation électrique 26 dissipe une puissance largement supérieure au flux thermique à mesurer, due à la combustion du gaz. On crée ainsi dans la cheminée d'évacuation 10 un courant de convection nautrel de débit pratiquement indépendant du flux thermique à mesurer. La cheminée 10 débouche dans la partie haute de la chambre 11, munie d'une tubulure 27 d'évacuation des gaz brûlés. La partie basse de la chambre, séparée de la partie haute par une cloison transversale 28, reçoit l'air de combustion par l'intermédiaire d'un humidificateur 29 et d'un détendeur 30. Les conditions d'emploi doivent être telles qu'il n'y ait pas de condensation dans la cheminée: on mesure ainsi le pouvoir calorifique inférieur.

Lorsque l'installation est utilisée pour mesurer le pouvoir calorifique d'un gaz naturel, il est souhaitable de disposer de moyens d'étallounage. Dans le mode de réalisation illustré en figure 1, ces moyens comportent une électrovanne 31 permettant d'alimenter le régulateur de pression 22 et le brûleur 9 alternativement en gaz combustible à étudier et en un gaz de référence, qui sera par exemple du méthane pur contenu dans une bouteille sous pression 32. La durée de chaque étalonnage sera évidemment déterminée par le temps nécessaire pour arriver à un nouvel équilibre thermique: elle sera en règle générale inférieure à une heure.

On peut utiliser, au lieu d'une alimentation alternative du brûleur 9, une résistance électrique chauffante permettant un étalonnage par effet Joule. Mais cette façon de procéder implique que l'encombrement et la forme de la résistance soient comparables à ceux du brûleur, de façon à créer un courant de convection naturel équivalent. Il faut de plus introduire un facteur de correction tenant compte du rayonnement différent de la résistance et du brûleur, même lorsque de brûleur est entouré d'un écran dont la température est voisine de celle de la résistance d'étalonnage.

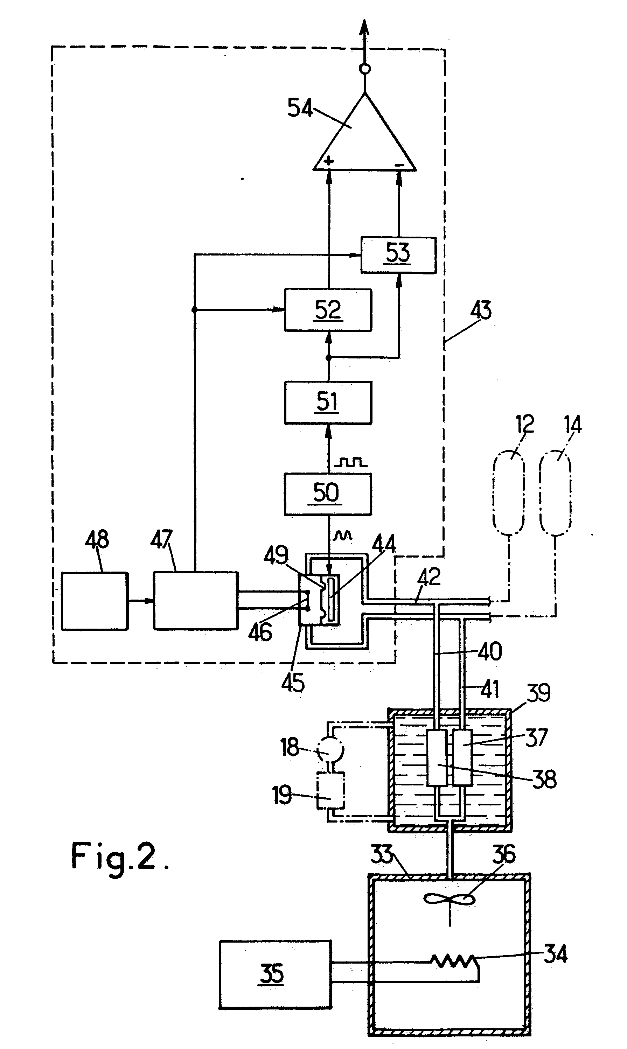

Comme on l'a indiqué plus haut, les enceintes 12 et 14 doivent être associées dans un ensemble de mesurer que l'on peut comparer à un thermomètre à gaz. Dans le cas particulier illustré sur la figure 2, les enceintes 12 et 14 sont associées à un pont pneumatique du type décrit dans la demande de brevet déposée ce jour au nom de l'ONERA et déjà mentionnée (FR-A-2 514 128). Ce pont de mesure pneumatique comporte une alimentation en gaz (qui peut être de l'air) sous un débit périodique alternatif. Dans le mode de réalisation montré en figure 2, ce débit est créé par dilatation thermique pulsée. Les moyens de pulsation comportent un réservoir 33 qui communique avec le pont et dans lequel est placée une résistance chauffante 34 associée à une alimentation électrique 35 fournissant des créneaux de courant. Un ventilateur 36 à fonctionnement permanent, placé dans le réservoir 33 à proximité de la sortie de celui-ci, maintient un écoulement d'air permanent sur la résistance 34 de façon à provoquer des variations de pression périodiques alternatives, qui sont en pratique approximativement sinusoïdales.

Le pont pneumatique proprement dit comporte deux éléments de perte de charge 37 et 38 dans lesquels un débit alternatif est créé par le réservoir 33 et respectivement placés en série avec les enceintes 12 et 14. Les éléments de perte de charge sont constitués par des blocs percés de conduits de diamètre suffisamment faible pour que l'écoulement y soit laminaire et donc que la perte de charge soit une fonction sensiblement linéaire de la vitesse moyenne de l'écoulement. A titre d'exemple, on peut indiquer que, dans un pont pneumatique dont les moyens de création de débit alternatif fonctionnement à une fréquence de l'ordre de 1 Hz, on a constitué les éléments de perte de charge par des barreaux de céramique de 1 cm de longueur, percés de quelques trous de 0,2 mm de diamètre. Pour que les pertes de charge subies par le gaz à la traversée des éléments 37 et 38 soient bien déterminées et constantes, la température de ces éléments et du gaz qui les traverse doit être également constante. Dans ce but, les éléments 37 et 38 sont placés dans une enceinte 39 occupée par un liquide dont la température est constante. L'enceinte 39 peut d'ailleurs être incorporée à l'enceinte 17 (figure 1) et utiliser la même pompe 18 et le même thermostat 19.

Une telle disposition permet d'obtenir par exemple une surpression moyenne de l'ordre du tiers de la pression atmosphérique, avec une modulation dont l'amplitude est de quelques dizaines de millibars.

Les éléments de perte de charge 37 et 38 sont avantageusement aussi identiques que possible. De même, il sera généralement préférable de donner aux enceintes 12 et 14 le même volume. Dans ces conditions, les tubulures 40 et 41 qui relient les éléments de perte de charge aux enceintes sont à la même pression lorsqu'aucun déséquilibre n'intervient entre les branches du pont.

Si au contraire un déséquilibre so produit, du fait d'un écart entre les températures moyennes des gaz contenus dans les enceintes 12 et 14, du fait du flux calorifique de la flamme du brûleur qui augmente la température du gaz dans l'enceinte 12 davantage que dans l'enceinte 14, un débit alternatif tend à circuler entre les enceintes 12 et 14, dans une canalisation 42 qui les relie. Le calcul montre que, aussi logtemps que la différence de température entre les enceintes 12 et 14 reste faible par rapport à la température moyenne de ces enceintes, l'écart de pression entre les extrémités de la conduite de liaison est proportionnel à l'écart de température entre les enceintes. Le calcul fait également apparaître que la réponse du pont est d'autant meilleure que la fréquence d'excitation, fixée par le générateur de créneaux 35, est plus proche d'une valeur optimale déterminable par le calcul, fonction des caractéristiques dimensionnelles du pont.

Dans le mode de réalisation montré en figure 2, la pression différentielle est mesurée à l'aide d'un microdébitmètre 43 du type décrit et revendiqué dans le brevet français n° 7 511 844, publié sous le n° 2 308 090, auquel on pourra se reporter. La constitution et le fonctionnement de ce microdébitmètre ne seront donc que sommairement décrits. Il comporte un capteur 44 dans lequel est ménagée une chambre 45 interposée sur la conduite 42. Dans cette chambre est placé un élément thermorésistant 46 de faible épaisseur placé dans un pont de mesure électrique 47. Le pont est alimenté par un circuit 48 en impulsions électriques de chauffage. A l'écoulement entre les enceintes 12 et 14 qui balaie l'élément thermorésistant 10, est superposé un écoulement alternatif, à une fréquence qui est d'au moins de deux ordres de grandeur supérieure à la fréquence des pulsations de pression fournies par le réservoir 33. Ces oscillations de pression sont réalisées par une membrane 49 excitée par une bobine. Cette dernière reçoit un signal sinusoïdal d'un oscillateur 50 qui fournit des créneaux carrés à la même fréquence et avec la même phase à un circuit de mesure comprenant un déphaseur 51. Les sorties de ce déphaseur attaquent les entrées de commande de deux mémoires 52 et 53 qui reçoivent les signaux de mesure provenant du pont. Les signaux mémorisés en mémoire 52 et 53 sont appliqués sur les deux entrées d'un amplificateur différentiel 54 dont la sortie fournit un signal de mesure.

On peut ainsi constituer une installation de mesure à faible temps de réponse (typiquement de l'ordre de 10 min), à faible consommation de combustible, permettant d'arriver à une précision élevée.

L'invention est susceptible de nombreuses variantes de réalisation. Par exemple, le brûleur peut être alimenté non pas à débit constant, mais à débit régulé, de façon que le flux thermique de la flamme soit constant. Le pouvoir calorifique est alors déduit du débit. Le mesure du flux thermique traversant le calorimètre peut être effectuée par d'autres moyens que ceux donnés à titre d'exemples plus haut.

Il doit être entendu que la portée du présent brevet ne se limite pas aux modes de réalisation qui ont été particulièrement décrits et représentés.

## Revendications

1. Installation de mesure en continu du pouvoir calorifique d'un gaz combustible, comportant un calorimètre dans lequel est placée une cellule ouverte (8) dont la partie basse entoure un brûleur (9) et des moyens (22, 30) alimentant le brûleur et la cellule respectivement en gaz combustible et en air de combustion à pression et débit déterminés, caractérisée en ce que le calorimètre comprend une paroi externe (16) maintenue à température constante et délimitant, avec la paroi de la cellule (8), un espace annulaire séparé, au moyen d'une cloison (13) parallèle aux parois, en une enceinte interne (12) et une enceinte externe (14) d'épaisseurs faibles par rapport à leurs autres dimensions, lesdites enceintes étant occupées par un même gaz et associées de façon à constituer un thermomètre différentiel à gaz.

2. Installation suivant la revendication 1, caractérisée en ce que la cellule comprend un tube sensiblement vertical muni de chicanes (24) et surmonté d'une cheminée d'évacuation (10) munie d'une résistance électrique chauffante dégageant une puissance largement supérieure au flux thermique à mesurer.

3. Installation suivant la revendication 1 ou 2, caractérisée en ce qu'elle comprend des moyens d'étalonnage permettant d'alimenter en alternance le brûleur en gaz combustible et en un gaz de référence.

4. Installation suvant l'une quelconque des revendications précédentes, caractérisée par des moyens pour alimenter de brûleur (9) à débit constant ou à débit régulé pour que le flux thermique traversant les enceintes soit constant.

5. Installation suivant l'une quelconque des revendications précédentes, caractérisée en ce que le calorimètre comporte un trajet unique de circulation d'air depuis les moyens de circulation d'air jusqu'à des moyens (27) de sortie d'air et de gaz de combustion.

6. Installation suivant l'une quelconque des revendications précédentes, caractérisée en ce que les enceintes (12, 14) sont associées à un circuit de mesure de la différence de pression entre les enceintes comportant un microdébitmètre monté entre les enceintes.

7. Installation suivant la revendication 6, caractérisée en ce que chacune des enceintes (12, 14) est reliée, au moyen d'élémentes (37, 38) imposant au gaz qui les traverse une perte de charge variant avantageusement suivant une fonction sensiblement linéaire du débit, à une alimentation pneumatique alimentant les deux enceintes en parallèle à partir des éléments de perte de charge et imposant un débit périodique alternatif.

8. Installation suivant la revendication 7, caractérisée en ce que les éléments de perte de charge (37, 38) sont constitués par des barreaux percés de plusieurs trous de faible diamètre et maintenus à température constante.

9. Installation suivant la revendication 8, caractérisée en ce que l'alimentation pneumatique est constituée par un réservoir (33) dans lequel est disposée une résistance chauffante (34) connectée à une alimentation électrique alternative (35).

**Patentansprüche**

1. Vorrichtung zur kontinuierlichen Messung des Heizwertes eines Brenngases mit einem Kaloriemeter, in welchem eine offene Zelle (8) angeordnet ist, deren unterer Bereich einen Brenner (9) umgibt, und mit Mitteln (22, 30), die den Brenner bzw. die Zelle mit Brenngas und mit Verbrennungsluft versorgen, mit einem vorbestimmten Druck und in einer vorbestimmten Menge, dadurch gekennzeichnet, daß das Kaloriemeter einen äußeren Schirm (16) aufweist, der auf einer konstanten Temperatur gehalten ist und mit dem Schirm der Zelle (8), ein getrennter Ringraum, und mit Hilfe einer Zwischenwand (13), die prallel zu den Wänden angeordnet ist, einen inneren Raum (12) und einen äußeren Raum (14) mit einer geringen Dicke in bezug auf deren anderen Dimensionen umgrenzt, wobei diese Räume mit einem gleichen Gas gefüllt und derart miteinander verbunden sind, daß sie ein Gasdifferenzialthermometer bilden.

2. Vorrichtung nach Anspruch 1, dadurch gekennzeichnet, daß die Zelle ein im wesentlichen vertikales Rohr aufweist, welches mit Schikanen (24) ausgerüstet ist und von einem Abzugskamin (10) überragt wird, der mit einer elektrischen Widerstandsheizung ausgerüstet ist, die eine Leistung entwickelt, die wesentlich höher ist, als die zu messende thermische Strömung.

3. Vorrichtung nach Anspruch 1 oder 2, dadurch gekennzeichnet, daß sie Eichmittel aufweist, die es erlauben, in wechselnder Folge den Brenner mit Brenngas und mit Referenzgas zu beschicken.

4. Vorrichtung nach einem der vorangehenden Ansprüche, gekennzeichnet durch Mittel zur Versorgung des Brenners (9) mit einer konstanten oder einer geregelten Menge zu versorgen, um den thermischen Fluß durch die Räume konstant zu halten.

5. Vorrichtung nach einem der vorangehenden Ansprüche, dadurch gekennzeichnet, daß das Kaloriemeter einen einzigen Zirkulationsweg der Luft von den Zirkulationsmitteln der Luft bis zu den Mitteln (27) des Austrittes von Luft und Verbrennungsgas aufweist.

6. Vorrichtung nach einem der vorangehenden Ansprüche, dadurch gekennzeichnet, daß die Räume (12, 14) mit einem Kreis zur Messung der Druckdifferenz zwischen den Räumen verbunden sind, der einen Mikrodurchflußmesser aufweist, welcher zwischen den Räumen eingebaut ist.

7. Vorrichtung nach Anspruch 6, dadurch gekennzeichnet, daß jeder der Räume (12, 14) mit Hilfe von Elementen (37, 38), die dem Gas, das sie durchströmt, einen Druckverlust aufzwingen, der vorteilhafterweise gemäß einer im wesentlichen linear zum Mengenbedarf verlaufenden Funktion variiert, mit einer pneumatischen Zuführung verbunden ist, die die beiden Kammern parallel auf der Grundlage der Druckverlustelemente versorgt und wechselweise periodisch einen Mengenbedarf aufbringt.

8. Vorrichtung nach Anspruch 7, dadurch gekennzeichnet, daß die Druckverlustelemente (37, 38) durch Stäbe gebildet werden, die von einer Vielzahl von Löchern geringen Durchmessers durchsetzt sind und auf einer konstanten Temperatur gehalten werden.

9. Vorrichtung nach Anspruch 8, dadurch gekennzeichnet, daß die pneumatische Zufuhr von einem Reservoir (33), in welchem eine Widerstandsheizung (34) untergebracht ist, die mit einer wechselnden elektrischen Versorgungseinrichtung (35) verbunden ist, gebildet ist.

**Claims**

1. Apparatus for the continuous measurement of the heating power of a fuel gas, comprising a calorimeter in which is located an open cell (8) whose lower portion surrounds a burner (9) and means (22, 30) for supplying the burner and the cell respectively with fuel gas and with combustion air at a predetermined pressure and feed rate, characterized in that the calorimeter comprises an external wall (16) maintained at a constant temperature and defining, with the wall of the cell (8), an annular space which is split by a partition (13) parallel to the walls into an internal enclosure (12) and an external enclosure (14) having thicknesses which are small as compared with their other dimensions, said enclosures being occupied by a same gas and being associated for constituting a differential gas thermometer.

2. Apparatus according to claim 1, characterized in that the cell comprises a substantially vertical duct provided with baffles (24) and topped by a discharge chimney (10) provided with an electric heating resistor delivering a power which is much greater than the thermal flux to be measured.

3. Apparatus according to claim 1 or 2, characterized in that it comprises calibration means for alternately supplying the burner with fuel gas and with a reference gas.

4. Apparatus according to any one of the preceding claims, characterized by means for feeding the burner with a constant flow rate or with a flow rate which is controlled for maintaining the thermal flux acrosse said enclosures at a constant value.

5. Apparatus according to any one of the preceding claims, characterized in that the calorimeter has a single air-flow path from the means for circulating air up to means (27) for exiting air and combustion gases.

6. Apparatus according to any one of the preceding claims, characterized in that the enclosures (12, 14) are associated with a circuit for measuring the pressure difference between the enclosures, comprising a microflow-meter connected between the enclosures.

7. Apparatus according to claim 6, characterized in that each of the enclosures (12, 14) is connected through elements for subjecting the flow gas across them to a head loss which advantageously varies according to a substantially linear function of the flow rate to a pneumatic source which feeds both enclosures in parallel from the head loss elements and delivering an alternating periodical flow rate.

8. Apparatus according to claim 7, characterized in that the head loss elements (37, 38) consist of rods formed with a plurality of passages of low diameter and maintained at a constant temperature.

9. Apparatus according to claim 8, characterized in that the pneumatic source comprises a tank (33) containing a heating electrical resistor (34) connected to an AC power source (35).

Fig.1.

Fig.2.